# EUROPEAN PATENT APPLICATION

(11) **EP 1 598 099 A1**
(43) Date of publication of application: **23.11.2005**
(21) Application number: 02796776.9
(22) Date of filing: 11.11.2002
(51) Int. Cl.: B01D 5/00, A61B 5/097, G01N 33/497, F25B 21/02

(54) **THERMOELECTRIC CONDENSER FOR VAPOUR EXHALED DURING RESPIRATION**

(30) Priority: 02.09.2002 ES 200202217 U
(71) Applicant: Romero Colomer, Pablo Vicente, 08859 Begues (ES); Colomer Garcia, Juan Carlos, 46001 Valencia (ES)
(72) Inventor: Romero Colomer, Pablo Vicente, 08859 Begues (ES); Colomer Garcia, Juan Carlos, 46001 Valencia (ES)
(74) Representative: Curell Aguilà, Marcelino
(86) International application number: PCT/ES2002/000522
(87) International publication number: WO 2004/020067

(57) **Abstract**

The objective of the present invention is to collect exhaled vapour for diagnostic purposes. The apparatus comprises:
I Alternating current to direct current transformer.
II Thermoelectric pump comprising thermoelectric plates stuck to the flat side of the ventilated radiator by means of heat conducting putty.
III Condensation nucleus, comprising the transmission part and the condensation tubes. The cooling surface of the thermoelectric plates is stuck to the surface of the transmission part, consisting of a metallic block that contains the condensation tubes. The condensation tubes are two glass cylinders which are introduced in the transmission part. The lower end of both tubes is joined with a Y piece that hermetically closes the circuit.
IV. The respiration circuit drives the air exhaled by the subject to the condensation tube, preventing re-inspiration by means of one-way valves.

## Description

### Objective:

Particles in suspension in the exhaled gas contain hydrosoluble molecules with low molecular weight from the bronco-alveolar surface of the lung. Some of these substances are bio-chemical mediators which can act as diagnostic markers for various bronco-pulmonary complaints (inflammation, infection, allergies, neoplasias, etc...) The continuous condensing and collection of the aerosol particles present in the exhaled gas is the objective of this invention. The apparatus is designed to be able to collect the exhaled condensate for an indefinite period of time both in subjects who breathe spontaneously and those on mechanical ventilation. The apparatus collects the exhaled aerosol by the cooling of a tube (condenser) in close contact with a metallic part which acts as support for the condenser tube and as the transmitter of a thermoelectric pump. The apparatus permits collection of the condensate in either liquid (condensation), or solid form (freezing) by varying the power of heat extraction.

### Background to the invention:

There are few apparatus available to condense vapour and the aerosol particles in exhaled breath. All of them use liquid refrigerants and heat extraction is obtained either with a closed circuit compressor, or by extemporaneous cooling of the refrigerant.
In the case of closed circuits with refrigerant the problem is the use of substances that are potentially aggressive for the environment and the need for a compressor. In the case of the liquids which are cooled extemporaneously, their effect is limited in time and the number of collections per instrument is limited, depending on the liquid used. The invention we propose allows unlimited collection of the condensate and uses a "clean" energy source (electric), with no potential environmental pollutants.
Furthermore, the instruments available only permit collection of the exhaled breath in open circuit, in other words the exhaled air goes to the atmosphere. Our invention makes it possible to fit the condenser into the espiratory tube of any mechanical ventilation equipment and thus permits the continuous collection of condensate in ventilated patients and in a closed circuit.

### Description of the invention:

The apparatus has five modules: I Supply source; II Thermoelectric pump; III Condenser; IV. Respiratory circuit and V. Support
I. The supply source is an a.c. to d.c. transformer, to feed the thermoelectric plates. The transformer is insulated and shortcircuitable and it is connected to the thermoelectric pump by a protected cable which makes it possible to physically separate the transformer from the condenser.
II. The thermoelectric pump is based on the peltier effect and consists of: a) a dissipator, which itself consists of one or more fans and a radiator and b) the thermoelectric plates and c) the transmission part. The thermoelectric plates (commercial ones) are stuck to the flat side of the radiator and contact is ensured with an adhesive gel or heat conducting putty. The surface of the thermoelectric plates away from the radiator is stuck to the outside of the transmission part, which is a mechanised metallic block and is in close contact with and attached to the condensation tubes. The transmission part is covered with heat insulation except for the surface in contact with the thermoelectric plates. The union between the thermoelectric plates and the transmission part is ensured with adhesive gel or heat conducting putty. The set: radiator-thermoelectric plate-transmission part are pressure joined. Temperature is controlled with a digital thermometer, the heat probe is introduced in an orifice in the transmission plate made for that purpose.
III. The condensation tube is composed of two glass or aluminium cylinders, depending on the application, which are positioned in parallel and vertically, attached to the transmission plate. The bottom end of both tubes is joined /closed with a part or collector, which closes the circuit by adjusting the pressure, hermetic, while at the top end of both tubes an adaptor can be place for a standard corrugated tube.
IV. Respiration circuit: It impulses the exhaled breath to the condensation tube, preventing re-inspiration by a set of one-way valves.
   Condensation in the respiration circuit is prevented by the fact that the circuit is heated. Direct connection of the patient to the respiration circuit varies depending on whether the patient is breathing spontaneously (mouth piece or mask) or undergoing mechanical ventilation. In the latter case, the condenser is placed in the espiratory branch of the ventilator's respiratory circuit.
V. The condenser will be attached to a support or workbench which will allow the work to be done in a vertical or almost horizontal position, by pivoting on an axis. Although the transmission part is effectively isolated from ambient air, a fin inserted in the longitudinal slots at the bottom of the radiators will make it possible to collect any condensation of ambient water which may occur and channel it towards a tray located underneath the apparatus.

Independent of the object of the invention are the number and dimensions of the thermoelectric plates, the configuration of the radiant element and the material in the radiant element and the heat transmission part. Also independent of the object of the invention is the configuration, dimensions and material in the respiration circuit, but only when re-inspiration of the exhaled gas is prevented.

### Brief description of the figures

Figure 1(a) Standard condenser set, installed on a bench, in horizontal position. 1: radiators; 2: fans; 3: space for heat insulation; 4: heat transmission block, in the space between this and the radiator, the thermoelectric plates are installed; 7:axis and supports for rotating the equipment and positioning it vertically or horizontally; 8: thermostat and switches. Figure 1 (b): the same apparatus but in vertical position, showing: 5: orifices for introducing the condensation tubes; and 6 orifice for introducing heat probe.

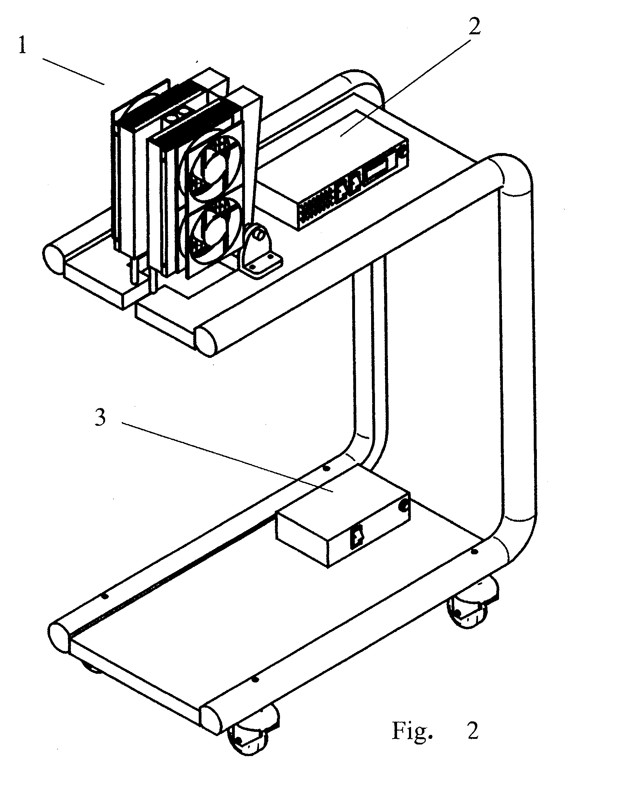

Figure 2: Condenser set installed on a table with wheels. 1: condenser as in Figure1; 2: thermostat and switches; 3: AC/CC transformer, power supply and on/off switch.

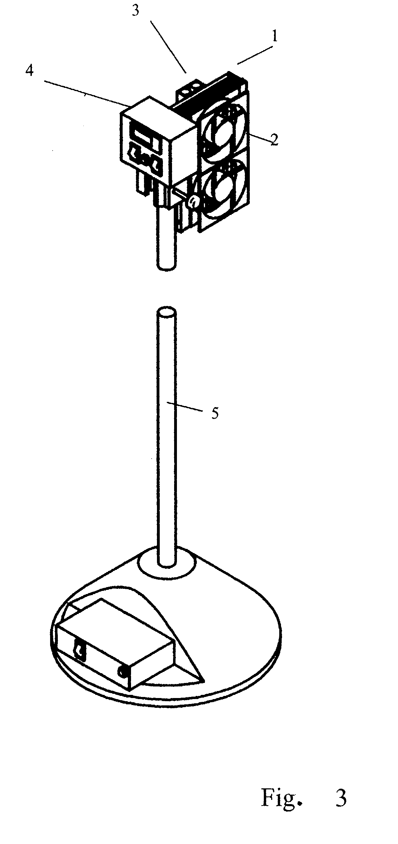

Figure 3.- Light condenser set mounted on a vertical stand (5). The figure shows the radiator (1) and the fans (2). The orifices for introducing the condensation tubes (5) and the thermostat (6). The back cover, covered with insulating material on the inside has not been drawn.

### Description of a preferred way of manufacture

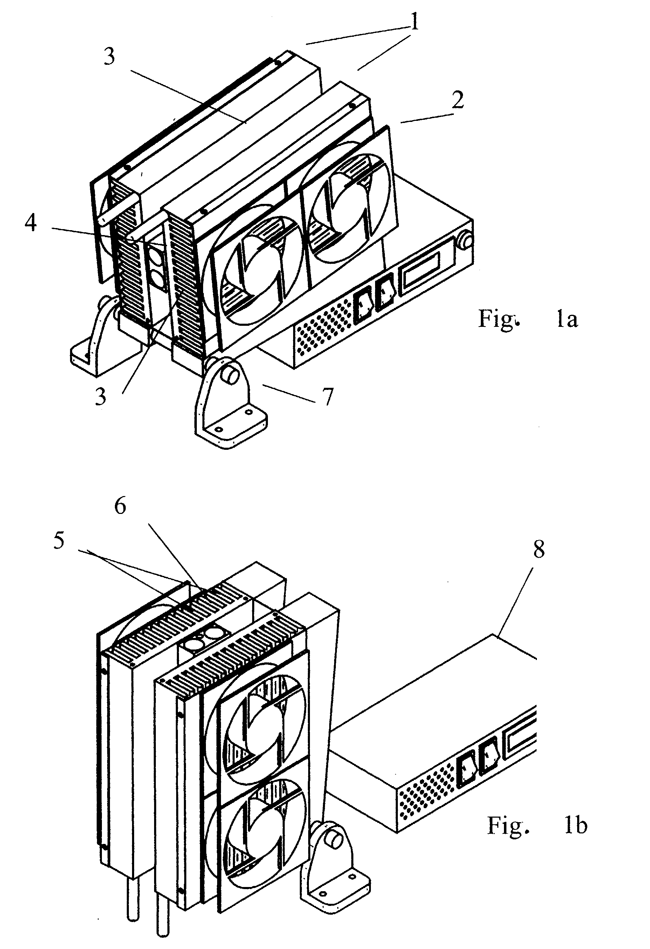

In accordance with the above description, we detail a characteristic configuration of the condenser, the motive of this invention. It consists of (Figure 1):
a) Two radiating elements comprising a passive radiant element (1) which we shall build in aluminium with a high radiation surface design and two to four active elements (2), (fans) which direct the air current towards the radiators.
b) Thermoelectric plates, minimum number of two, approximately 4 x 4 cm, vertically placed between the radiators and the transmission part.
   The whole set is firmly fixed with screws and the virtual spaces between the conducting surfaces (radiator-plate-transmitter) filled with heat conducting putty. The intermediate empty space around the thermoelectric plates (3) is filled with a common heat insulator (rubber mould or blanket) which also acts to seal the space occupied by the plates to prevent condensation on them.
c) The transmission part (4) is in the shape of an aluminium prism with two parallel orifices running through it in a longitudinal direction (5). The dimensions of the block depend on the application. For a standard application (18-20 mm collecting tubes), the dimensions are approximately 200×80×30 mm. The orifices have a tolerance of +10% with respect to the external diameter of the condensation tube, which allows it to be introduced. A toric ring near to one of the ends of the tube limits the extent to which it can be introduced. The condensation tubes are glass tubes of variable diameters according to the application (standard 18-20 mm Øₑₓₜ for an adult). The bottom ends of the tubes are closed with a plastic Y piece or collector, which adjusts the pressure in the tubes. In the bottom orifice of the collector, a collection tube is installed. The set is introduced in the orifices of the transmission part where it is attached by the torico rings
d) Temperature control is established with a digital thermometer-thermostat with a probe which is placed inside an orifice made for that purpose (6) in the centre of the metallic transmission plate, between the two collection tubes. The temperature is read on a digital screen on the cover of the apparatus. The subject can, at will, limit the working temperature.

The set will be covered by a fixed casing that will have directional fins to direct the air away from the subject. A digital thermometer will permit the temperature of the block to be read and optionally thermostatised. The back of the covering will have a tray to collect the condensed water from the block and allow cleaning.

The respiration circuit is variable according to the application with subjects breathing spontaneously (open circuit) or subjects on mechanical ventilation (closed circuit). In the case of subjects breathing spontaneously, on the upper part of one of the tubes, a standard ventilator tube is connected, covered with a CC electric blanket fed from the power supply through the condenser where there is an electrical power point. The tube is connect at one end to the condenser tube and at the other to one of the branches of a Y or T part with a one-way inspiratory valve on the free branch. At the upper end of the second condensation tube there is a one-way expiratory valve. The subject breathes into the Y or T piece by the mouth using a commercial rubber mouth piece or mask, the nose can be occluded using a low pressure nose peg.

In the case of the insertion of the condenser in the expiratory circuit of a mechanical ventilator, the heated corrugated tube is connected to the expiratory branch of the Y or T piece in the ventilator's respiration circuit and at the upper end to one of the condensation tubes, while the expiratory tube of the ventilator is connected to the upper end of the other condensation tube.

## Claims

1. Thermoelectric condenser of exhaled breath vapour, comprising the following elements: I: Power supply; II Thermoelectric pump; III Condensation tube and IV Respiration circuit: I The power supply consists of an alternating current to direct current transformer, to supply the thermoelectric plates. II The thermoelectric pump is based on the peltier effect and consists of: a) a disipator, comprising one or more fans and a radiator and b) thermoelectric plates. The thermoelectric plates are stuck to the flat side of the radiator and contact is ensured by heat conducting putty. The surface of the thermoelectric plates away from the radiator are stuck to the internal side of the transmission part in the form of a metal block which contains the condensation tubes. The union between the thermoelectric plates and the transmission part is ensured by heat conducting putty. Temperature control is established by a digital thermometer, the heat probe of which is introduced in an orifice in the transmission part made for that purpose. III The condensation tube comprises two glass cylinders that are positioned in parallel and vertically, inside the transmission part. The lower end of both tubes is joined using a piece or collector, which closes the circuit by adjusting the pressure, hermetic, while at the upper end of both tubes it is possible to fix an adaptor for a standard corrugated tube. IV. Respiration circuit: Drives the air exhaled by the subject towards the condensation tube, preventing re-inspiration by means of one-way valves. Condensation in the respiration circuit is avoided by thermostat controlled heating thereof. The direct connection of the subject to the respiration circuit varies according to whether the subject is breathing spontaneously (mouth piece or mask) or is on mechanical ventilation. In the latter case, the condenser apparatus is placed in the expiratory branch of the respiratory circuit of the ventilator.

2. Less bulky condensation equipment consisting of half of the described condenser device (figure 2), comprising a ventilated radiator, two or three thermoelectric plates and the transmission part, covered with insulating material. A cover external to the transmission part and fixed to the radiator ensures sufficient elastic pressure to maintain a firm contact between the thermoelectric plate and the transmission part. A pressure device allows fixation of the condenser to a vertical stand with wheels or a drip support, for transport to the patient's bedside.

3. Design described in claim 1, provided with a suction pump to condense vapour from the atmosphere of a ventilated area, in which there is production of vapour of animal origin (cages or controlled atmosphere areas), vegetable origin (nurseries, greenhouses, etc.) or other origins (broths of biological cultures).

4. Any of the previous designs, utilising for refrigeration of the thermoelectric pump one or several circulating water radiators or circulating refrigeration liquid radiators.

## Amended claims

### Amended claims under Art. 19.1 PCT

**1.** 1. Thermoelectric condenser of exhaled breath vapour, comprising the following elements: I: Power supply; II Thermoelectric pump; III Condensation tube and IV Respiration circuit: I The power supply consists of a AC/CC transformer, to supply the thermoelectric plates. II The thermoelectric pump is based on the peltier effect and consists of:
a) a disipator, comprising one or more fans and a radiator and b) thermoelectric plates. The thermoelectric plates are stuck to the flat side of the radiator and contact is ensured by heat conducting putty. The surface of the thermoelectric plates away from the radiator are stuck to the internal side of the heat transmission part in the form of a metal block which contains the condensation tubes. The union between the thermoelectric plates and the transmission piece is ensured by heat conducting putty. Temperature control is established by a digital thermometer, the heat probe of which is introduced in an orifice in the transmission part made for that purpose. III The condensation tube comprises two glass cylinders which are positioned in parallel and vertically, inside the transmission part. The lower end of both tubes is joined using a piece or collector which closes the circuit by adjusting the pressure, hermetic, while at the upper end of both tubes it is possible to fix an adaptor for a standard corrugated tube. IV. Respiration circuit: Drives the exhaled air towards the condensation tube, preventing re-inspiration by means of one-way valves. The direct connection of the subject to the respiration circuit varies according to whether the subject is breathing spontaneously (mouth piece or mask) or is on mechanical ventilation. In the latter case, the condenser is placed in the expiratory branch of the respiratory circuit in the ventilator.

**2.** As claim 1, but varying the number of condensation tubes (more than two) joined in the form of a serpentine at the ends, with a common collector for all of them in the lower part.

**3.** As the two previous claims, but with metallic cylinders instead of glass ones.

**4.** As the above claims, but using cylinders made from plastic

**5.** As the above claims, but replacing the thermometer with an adjustable thermometer-thermostat, to limit the cooling temperature by acting (connection-disconnection) on the power supply to the peltier plates.

**6.** As claim 1, but incorporating a power regulator to adjust the strength of supply to the peltier plates, to modify heat strength.

**7.** As claim 1, but using multistage (or in cascade) peltier plates to increase cooling power.

**8.** As the above claims, but using radiators cooled by cooling liquid circulating inside.

**9.** As claim 8, but maintaining simultaneously air refrigeration of the radiators.

**10.** As the above claims, but with two thermoelectric pumps, one on each side of the heat transmission block

**11.** As claim 10, but replacing the heat transmission block with two longitudinal hemisections of the same block cut in the diametral plane common of the orifices for the condensation tubes. These are trapped using a mechanical system to join the hemisections.

**12.** As the above claims, but heating, regulated by thermostat, the respiration circuit, to avoid condensation in the circuit.
